# FASCICULE DE BREVET EUROPEEN

(11) **EP 1 896 016 B1**
(45) Date de publication et mention de la délivrance du brevet: **11.08.2010**
(21) Numéro de dépôt: 06778604.6
(22) Date de dépôt: 16.06.2006
(51) Int. Cl.: A61K 31/4164, A61K 9/08, A61K 47/22, A61P 17/00

(54) **PROCEDE DE SOLUBILISATION DU METRONIDAZOLE**
VERFAHREN ZUR SOLUBILISIERUNG VON METRONIDAZOL
METHOD FOR SOLUBILIZING METRONIDAZOLE

(30) Priorité: 17.06.2005 FR 0506182
(43) Date de publication de la demande: 12.03.2008
(60) Demande divisionnaire: 10160846.1
(73) Titulaire: Galderma S.A., 6330 Cham (CH)
(72) Inventeur: MALLARD, Claire, F-06250 Mougins (FR); BRZOKEWICZ, Alain, F-06600 Antibes (FR)
(74) Mandataire: Allab, Myriam
(86) Numéro de dépôt international: PCT/FR2006/001367
(87) Numéro de publication internationale: WO 2006/134279

(56) Documents cités:
- WO-A-02/06349
- WO-A-02/094179
- WO-A-03/057135
- US-A- 4 032 645
- CHIEN Y W: "SOLUBILIZATION OF METRONIDAZOLE BY WATER-MISCIBLE MULTI-COSOLVENTS AND WATER-SOLUBLE VITAMINS" JOURNAL OF PARENTERAL SCIENCE AND TECHNOLOGY, vol. 38, no. 1, janvier 1984 (1984-01), pages 32-36, XP002947918 ISSN: 0279-7976 cité dans la demande
- PATENT ABSTRACTS OF JAPAN vol. 007, no. 147 (C-173), 28 juin 1983 (1983-06-28) & JP 58 059913 A (WAKOUDOU KK), 9 avril 1983 (1983-04-09)

## Description

La présente invention concerne le domaine de la formulation de principe actif en vue d'applications pharmaceutiques, notamment pour la voie topique.

La présente invention concerne en particulier un nouveau procédé de solubilisation du métronidazole au sein d'une composition, associant en phase aqueuse, au moins du métronidazole, de la niacinamide, du propylène glycol et un polyéthylène glycol. Cette association permet de solubiliser le métronidazole dans ladite composition.
L'invention concerne également les solutions issues du procédé de solubilisation selon l'invention, leur méthode de préparation, les compositions résultantes contenant le métronidazole ainsi solubilisé, et leur utilisation en médecine humaine ou vétérinaire.

Le métronidazole, ou 1-(2-hydrokyéthyl)-2-méthyl-5-nitroimidazole, est depuis longtemps connu comme produit actif dans le traitement d'affections diverses, et en particulier dans le traitement de maladies dues à divers Protozoaires. En application topique, le métronidazole est également utilisé dans le traitement d'affections dermatologiques diverses, dont la rosacée *(acne rosacae)*, les ulcères bactériens, et la dermatite *periorale*, comme décrit dans le brevet US 4 837 378. Le métronidazole montre également une activité anti-inflammatoire lorsqu'il est utilisé topiquement dans le traitement de troubles dermatologiques, comme décrit dans le brevet US 5 849 776. Le métronidazole peut aussi être utilisé dans le traitement de *vaginosis* bactérien, comme agent thérapeutique intravaginal, comme décrit dans le brevet US 5 536 743.

Les compositions contenant le métronidazole pour le traitement de désordres dermatologiques sont disponibles sous forme de crème, de gel ou de lotion. Le NORITATE™ (Dermik Laboratories, Inc.) contient 1% de métronidazole dispersé dans une crème blanche. Galderma Laboratories, Inc propose le MetroGel ®, contenant 0.75% en masse par rapport à la masse totale de la composition (m/m) de métronidazole solubilisé dans un gel transparent, le MetroCream ® contenant 0.75% (m/m) de métronidazole solubilisé au sein d'une crème émolliente, et MétroLotion ® contenant 0.75% (m/m) de métronidazole solubilisé au sein d'une lotion.

Dans le cadre des applications topiques, les produits contenant le principe actif sous forme solubilisée présentent souvent une meilleure biodisponibilité que les produits dans lesquels le principe actif est dispersé.

Etant donné la faible solubilité intrinsèque du métronidazole en phase aqueuse, qui est de l'ordre de 0,9% (m/m), les compositions aqueuses gélifiées actuellement disponibles sur le marché sont limitées à une concentration de 0,75% (m/m) en métronidazole, solubilisée dans la formulation. Les formulations du métronidazole sous forme de crèmes présentent l'avantage, par rapport aux formulations gélifiées actuellement disponibles, de contenir 1 % (m/m) de métronidazole.

D'une manière générale, la solubilité des principes actifs en phase aqueuse peut être augmentée en utilisant des tensio-actifs divers, non ioniques, ioniques, des dérivés lipidiques comme des lécithines permettant la formation de micelles lipidiques ou des cyclodextrines comme souvent décrit dans la littérature.
Les cyclodextrines permettent d'augmenter la solubilité dans l'eau de divers composés. Les cyclodextrines se présentent sous forme de cage. La partie externe hydrophile de cette cage leur confère une certaine solubilité dans les milieux aqueux. Leur partie interne, plus hydrophobe permet la solubilisation de molécules amphiphiles ou lipophiles, par la formation de complexes d'inclusion. La solubilité dans l'eau de nombreuses molécules peut donc être sensiblement augmentée par l'utilisation de ces cyclodextrines. Cependant, les cyclodextrines présentent des inconvénients de coûts, de solubilité limitée, d'incompatibilté avec certains véhicules ou excipients, ou de toxicité locale ou systémique potentielle. D'autre part, la formation des complexes d'inclusion, étape de complexation, peut être assez longue, souvent plusieurs heures, et avoir des incidences sur les coûts et procédés de fabrication des formulations.

Des agents augmentant la solubilité autres que les cyclodextrines ont été décrits. Yie W. Chien, Journal of Parenteral Science and Technology, 38 (1) :32-36 (Jan. 1984), montre que la solubilité du métronidazole en solution aqueuse peut être augmentée à l'aide de vitamines hydrosolubles comme la niacinamide, la pyridoxine hydrochloride et l'acide ascorbique. Chien décrit ensuite que la solubilité du métronidazole dans l'eau augmente de façon linéaire en relation avec la concentration de ces vitamines hydrosolubles en solution. En particulier, Chien montre qu'il est nécessaire d'avoir au moins 9 moles de niacinamide en solution pour solubiliser une mole de métronidazole. En particulier, Chien a montré qu'une combinaison de niacinamide avec du métronidazole selon un ratio molaire 9 pour 1 permet d'augmenter la solubilité en phase aqueuse du métronidazole. Ainsi pour obtenir une formulation contenant 1 % (m/m) en métronidazole solubilisé dans la phase aqueuse, il est nécessaire d'introduire une forte teneur en niacinamide de l'ordre de 6,4% (m/m).

Y.Chang, G.dow et al. ont protégé au sein du brevet US 6,468,989, sur la base des travaux de Chien et al., l'utilisation de cyclodextrines en combinaison avec la niacinamide afin de formuler des gels de métronidazole à 1 % (m/m) avec une teneur en niacinamide inférieure à celle de l'art antérieur, à savoir de 1,25% (m/m) seulement. Ils ont démontré un effet synergique de l'association niacinamide / cyclodextrines sur la solubilité du métronidazole.

De façon surprenante, la demanderesse est parvenue, dans la présente invention, à solubiliser le métronidazole en absence des solvants classiquement utilisés dans la littérature. Plus précisément, la demanderesse a solubilisé le métronidazole en absence de cyclodextrine ou de tensio-actif, et en présence d'une faible teneur en niacinamide.

Le but de la présente invention est de proposer un procédé nouveau permettant de solubiliser le métronidazole en phase aqueuse. La présente invention concerne donc un procédé de solubilisation du métronidazole au sein d'une composition exempte de cyclodextrine ou de tensioactif et comprenant une faible teneur en niacinamide.

La présente invention a également pour objet une solution aqueuse comprenant le métronidazole, la niacinamide, au moins deux co-solvants glycoliques, tels que le propylène glycol et un polyéthylène glycol, et de l'eau.

Selon un troisième aspect, la présente invention a pour objet une composition pharmaceutique comprenant la solution selon l'invention.

Selon un quatrième aspect de l'invention, celle-ci a pour objet l'utilisation d'une solution ou d'une composition pharmaceutique selon l'invention telles que définies précédemment pour la fabrication d'un médicament destiné au traitement d'une affection dermatologique, notamment la rosacée, l'acné vulgaire ou la dermatite séborrhéique, et préférentiellement de la rosacée.

Le procédé selon l'invention permet une solubilisation du métronidazole en phase aqueuse à une concentration supérieure ou égale à la concentration de 0,75 % en masse par rapport à la masse totale de la solution (m/m) habituellement rencontrée dans les formulations commercialisées et plus particulièrement à une concentration supérieure ou égale à 1 % (m/m).

Le procédé de solubilisation du métronidazole selon l'invention comprend donc l'étape qui consiste à mélanger en phase aqueuse le métronidazole avec de la niacinamide, notamment en faible quantité, et des composés solvants glycoliques, de préférence au moins deux, en absence de cyclodextrine et/ou de tensioactif.

Par phase aqueuse, on entend une phase composée majoritairement d'eau.

Par solubilisation du métronidazole, on entend une dispersion à l'état moléculaire dans un liquide, aucune cristallisation du métronidazole n'étant visible à l'oeil nu ni même au microscope optique en polarisation croisée.

Selon l'invention, par métronidazole, on entend le métronidazole en tant que tel, mais aussi sous la forme d'un sel avec un acide pharmaceutiquement acceptable, ou encore sous forme d'un ester. Comme sel, on peut citer notamment le métronidazole hydrochloride. Par esters, on entend notamment l'acétate, le benzoate, le myristate, ou encore le monosuccinate de métronidazole.

Selon la présente invention, la niacinamide est utilisable en tant que telle, ou bien sous forme de sels avec un acide pharmaceutiquement acceptable.

De préférence, le rapport molaire niacinamide / métronidazole utilisé dans le procédé selon l'invention est strictement inférieur à 9, et préférentiellement inférieur ou égal à 5,5.

Selon la présente invention et de façon surprenante, la demanderesse a effectivement démontré que l'association de 2 cosolvants glycoliques en présence de niacinamide permet d'augmenter la solubilité du métronidazole en phase aqueuse.

Parmi les solvants glycoliques utilisables selon la présente invention, on peut citer notamment le Propylène glycol, le butylène glycol, l'éthoxydiglycol, le butoxydiglycol et les polyéthylène glycols (PEG).
Plus particulièrement les cosolvants glycoliques sont le Propylène glycol et au moins un polyéthylène glycol (PEG). Les PEG préférés sont les PEG liquides, comme les PEG 200 ; 300 ; 400 ; et 600.

Avantageusement, une association de 2 cosolvants glycoliques est utilisée, qui consiste en une association de Propylène glycol avec un polyéthylène glycol (PEG). Les 2 cosolvants préférés à utiliser en association selon la présente invention, sont le propylène glycol et le PEG 400.

En effet, comme le montrent les résultats présentés dans les exemples 1 et 2, dans la combinaison selon l'invention, le propylène glycol, le PEG 400 et la niacinamide agissent en synergie pour augmenter la solubilité aqueuse et la stabilité physique des solutions de métronidazole. De plus, la combinaison propylène glycol / PEG 400 permet de diminuer considérablement le ratio molaire niacinamide / métronidazole.

De façon préférée selon la présente invention, cette augmentation de solubilité du métronidazole en phase aqueuse est obtenue par l'association de 2 cosolvants, de préférence propylène glycol / PEG 400, selon un ratio massique de 1:1 en présence de niacinamide. Selon un mode préféré de réalisation, le ratio molaire niacinamide / métronidazole peut être diminué à 4. Selon un mode encore préféré de réalisation, la combinaison selon l'invention permet de solubiliser le metronidazole au moins jusqu'à une concentration de 1,56% (m/m) tout en ayant un ratio molaire niacinamide / métronidazole strictement inférieur à 9.

De façon avantageuse, le procédé de solubilisation du métronidazole selon l'invention comprend notamment les étapes suivantes :
a) préparation d'une solution de niacinamide, notamment à 10% (m/m), dans de l'eau, jusqu'à dissolution totale de la niacinamide. Une quantité de cette solution est utilisée, quantité qui dépend, comme vu précédemment, de la quantité de métronidazole à dissoudre et du rapport molaire niacinamide / métronidazole préalablement défini,
b) introduction dans la solution obtenue en a) des cosolvants glycoliques, de préférence au nombre de deux,
c) après homogénéisation de la solution obtenue en b), ajout d'une quantité définie de métronidazole. Cette quantité est fonction du rapport molaire niacinamide / métronidazole prélablement défini,
d) après dissolution totale du métronidazole, filtration de la solution obtenue.

Le métronidazole est ensuite dosé afin de vérifier le pourcentage solubilisé.

Préférentiellement, les deux co-solvants utilisés pour la solubilisation selon l'invention sont le propylène glycol et le PEG400.

De façon préférée et nullement limitative, le mélange se fait par agitation magnétique, la filtration sur filtre de 1µm et le dosage du métronidazole par mesure de densité optique par spectrophotométrie UV à la longueur d'onde de 327nm.

Sans cyclodextrine, la technique de solubilisation selon l'invention est plus avantageuse car ne nécessite pas d'étape de complexation.

Sans tensioactif nécessaire à la solubilisation, la composition contenant le métronidazole ainsi solubilisé, présente avantageusement un risque diminué d'irritations ou d'allergies cutanées.

L'invention concerne également la solution aqueuse de métronidazole susceptible d'être obtenue selon le procédé précédemment défini.

La solution aqueuse selon l'invention est **caractérisée en ce qu**'elle comprend :
- du métronidazole,
- de la niacinamide,
- au moins 2 co-solvants glycoliques, de préférence 2, qui sont de préférence le propylène Glycol et un polyéthylène glycol,
- de l'eau.

Une telle solution, comprenant le métronidazole sous forme solubilisée, ne contient notamment pas de cyclodextrine.

Dans la solution selon l'invention, le métronidazole est présent à une concentration supérieure ou égale à 0.75 % (m/m), préférentiellement supérieure ou égale à 1% (m/m).

La niacinamide dans la solution selon l'invention, est présente à une concentration telle que le rapport molaire niacinamide / métronidazole est strictement inférieur à 9, préférentiellement inférieur ou égal à 5,5.

Selon l'invention, on utilise de préférence deux co-solvants glycoliques. L'ensemble des deux co-solvants glycoliques, de préférence le propylène Glycol et un polyéthylène glycol, totalise une concentration comprise entre 2 et 50% (m/m), de préférence entre 5 et 20% et plus préférentiellement égale à 10%(m/m), étant entendu qu'ils sont présents dans un rapport massique de 1 :1. De façon préférée selon l'invention, le propylène glycol et le polyéthylène glycol sont présents dans un rapport massique de 1 :1, à la concentration de 5% (m/m) chacun.

De préférence, la solution selon l'invention comprend toutes les caractéristiques suivantes:
- du métronidazole en proportion supérieure ou égale à 1 %,
- de la niacinamide en proportion telle que le rapport molaire niacinamide / métronidazole est strictement inférieur à 9, et préférentiellement inférieur ou égal à 5,5,
- du propylène glycol et un polyéthylène glycol dans un rapport massique de 1 :1, le polyéthylène glycol étant préférentiellement le PEG 400.

La présente invention a également pour objet une composition pharmaceutique comprenant la solution selon l'invention. Une telle composition comprend le métronidazole en quantité supérieure ou égale à 0,75% en poids par rapport au poids total de la composition, de préférence supérieure ou égale à 1% en poids par rapport au poids total de la composition.

Selon la présente invention, on entend par composition, une composition pharmaceutique plus particulièrement destinée au traitement de la peau et des muqueuses et pouvant se présenter sous forme liquide, pâteuse, ou solide, et plus particulièrement sous forme d'onguents, de crèmes, de laits, de pommades, de poudres, de tampons imbibés, de syndets, de solutions, de lotions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

L'invention concerne également la solution et/ou la composition comprenant le métronidazole ainsi solubilisé, à titre de médicament.

Plus particulièrement, l'invention concerne l'utilisation de la solution et/ou de la composition comprenant le métronidazole ainsi solubilisé, pour la fabrication d'un médicament destiné au traitement d'une affection dermatologique, notamment la rosacée, l'acné vulgaire ou la dermatite séborrhéique. De façon préférée, l'affection dermatologique est la rosacée.

L'invention concerne enfin l'utilisation d'une association de niacinamide et d'au moins deux co-solvants glycoliques pour solubiliser le métronidazole dans une composition comprenant une phase aqueuse substantiellement exempte de cyclodextrine.
Par phase aqueuse substantiellement exempte de cyclodextrine, on entend une phase aqueuse qui ne contient pas de cyclodextrine.

Ci-après sont présentés, de façon non exhaustive, des exemples de solution selon l'invention, le procédé d'obtention de solutions de métronidazole, des résultats de stabilité ainsi que des exemples de composition contenant la solution selon l'invention.

### Exemple 1 : Préparations des solutions

Dans cet exemple, tous les essais de solubilité du métronidazole sont réalisés à partir d'une solution de niacinamide à 10% (m/m).

### Cas des solutions à 1% (m/m) en métronidazole sans cosolvant : (solutions 1 à 3)

La teneur en métronidazole est fixée à 1 % (m/m).
En fonction du rapport molaire fixé niacinamide / métronidazole, une quantité de solution à 10% en niacinamide est pesée et est ajoutée à la quantité de métronidazole pesée préalablement, puis l'ensemble est complété à 100% par addition d'eau distillée. Après 12 heures d'agitation magnétique, la solution obtenue est filtrée sur 1µm et dosée par UV à la longueur d'onde de 327 nm.

### Cas des solutions à 1% (m/m) en métronidazole avec cosolvant : (solutions 4 à 6)

La teneur en métronidazole est fixée à 1% (m/m) ainsi que le rapport molaire niacinamide / métronidazole à 4.
A la quantité de solution de niacinamide 10% (m/m) correspondante au ratio molaire préalablement défini, est ajouté, selon le cas, du propylène glycol ou du PEG 400 seuls ou en association. Le métronidazole est ensuite pesé et ajouté à la solution obtenue. Une quantité d'eau est ensuite ajoutée pour compléter à 100%.
Après 12 heures d'agitation magnétique, la solution obtenue est filtrée sur 1µm et dosée par UV à la longueur d'onde de 327 nm.

### Cas des solutions saturées en métronidazole : (solutions 7 et 8)

La teneur en métronidazole est fixée à 2% (m/m).
A la quantité de principe actif préalablement pesée est ajoutée le propylène glycol et le PEG400 selon un ratio massique de 1:1. Selon le cas, le niacinamide en solution 10% est ajouté de manière à respecter le ratio molaire niacinamide / métronidazole de 4. Ensuite, une quantité d'eau nécessaire est ajoutée pour compléter à 100%. Après 12 heures d'agitation magnétique, la solution obtenue est filtrée sur 1 µm et dosée par UV à la longueur d'onde de 327 nm.
Les solutions suivantes ont donc été préparées :

**TABLEAU I**

| **Solutions** | **Composition** | **Teneur(% m/m)** | **Rapport molaire Niacinamide / Métronidazole** | **Concentration mesurée (% m/m)** |
|---|---|---|---|---|
| | Métronidazole | 1,0 | | |
| **N°1** | Niacinamide 10% | 71,3 | 10 | 1,016 |
| | Eau distillée | 27,7 | | |
| | Métronidazole | 1,0 | | |
| **N°2** | Niacinamide 10% | 57,0 | 8 | 0,989 |
| | Eau distillée | 42,0 | | |
| | Métronidazole | 1,0 | | |
| **N°3** | Niacinamide 10% | 28,5 | 4 | 1,023 |
| | Eau distillée | 70,5 | | |
| | Métronidazole | 1,0 | | |
| **N°4** | Niacinamide 10% | 28,5 | 4 | 1,172 |
| | PEG 400 | 5,0 | | |
| | Eau distillée | 65,5 | | |
| | Métronidazole | 1,0 | | |
| **N°5** | Niacinamide 10% | 28,5 | 4 | 1,185 |
| | Propylène glycol | 5,0 | | |
| | Eau distillée | 65,5 | | |
| | Métronidazole | 1,0 | | |
| | Niacinamide 10% | 28,5 | 4 | 1,03 |
| **N°6** | Propylène glycol | 5,0 | | |
| | PEG 400 | 5,0 | | |
| | Eau distillée | 60,5 | | |
| | Métronidazole | 2,0 | | |
| **N°7** | Propylène glycol | 5,0 | NA | 0,998 |
| | PEG 400 | 5,0 | | |
| | Eau distillée | 88,0 | | |
| | Métronidazole | 2,0 | | |
| **N°8** | Niacinamide 10% | 57,0 | 4 | 1,565 |
| | Propylène glycol | 5,0 | | |
| | PEG 400 | 5,0 | | |
| | Eau distillée | 31,0 | | |

| | | | | |
|---|---|---|---|---|
| - : pas de données | | | | |

### Exemple 2 : Etude de stabilité

Les solutions préparées selon l'exemple 1 ont été suivies en stabilité sur une période de 8 semaines à température ambiante et à +4°C. Cette dernière condition étant plus drastique, elle permet de mettre en évidence plus rapidement les phénomènes de recristallisation potentiels du métronidazole. Les résultats obtenus sont les suivants :

**TABLEAU II**

| **Solutions** | **Stabilité à +4°C (observation visuelle)** | **Stabilité à température ambiante (observation visuelle)** |
|---|---|---|
| **N°1** | Limpide | Limpide |
| **N°2** | Précipitation à 13 jours | Limpide |
| **N°3** | Précipitation à 5 jours | Limpide |
| **N°4** | Précipitation à 8 jours | Précipitation à 18 jours |
| **N°5** | Précipitation à 8 jours | Précipitation à 18 jours |
| **N°6** | Limpide | Limpide |
| **N°7** | Précipitation à 8 jours | Précipitation à 8 jours |
| **N°8** | Limpide | Limpide |

### Conclusions :

La comparaison des solutions 1 et 2 a permis de confirmer les résultats de Chien et al. montrant qu'il est nécessaire d'introduire de la niacinamide dans les solutions aqueuses de métronidazolé pour augmenter sa solubilité et sa stabilité physique en phase aqueuse. Le rapport molaire niacinamide / métronidazole doit être supérieur à 8 afin d'obtenir une solution à 1% (m/m) en métronidazole stable physiquement pendant 8 semaines à température ambiante et à +4°C.

La comparaison des solutions 3 ; 4 ; 5 et 6 montre qu'il est nécessaire d'introduire le propylène glycol et le PEG 400 en combinaison selon un ratio massique préférentiel de 1:1, afin d'obtenir une solution stable pendant 8 semaines à +4°C et à température ambiante. Grâce à cette association, le rapport molaire niacinamide / métronidazole a pu être diminué à 4.

La comparaison des solutions 7 et 8 montre la nécessité d'introduire de la niacinamide dans le mélange ternaire eau / propylène glycol / PEG 400 afin d'obtenir une solution stable sur une période de 8 semaines à +4°C et à température ambiante.

La présence de la niacinamide dans une solution saturée en métronidazole permet d'augmenter la solubilité du principe actif au moins jusqu'à 1,5% (m/m) sans recristallisation (solution 8).

En complément des travaux de Chien et al., cette étude montre également que le ratio molaire niacinamide / métronidazole initialement de 9 peut être diminué d'un facteur 2 en présence de propylène glycol et de PEG 400 associés préférentiellement selon un ratio massique de 1:1.

Les résultats de cette étude permettent donc d'envisager la formulation du Métronidazole solubilisé à 1 % (m/m) avec une teneur en niacinamide très inférieure à 6,4% (m/m) sans l'utilisation de cyclodextrines ni de tensio-actifs supplémentaires dans la composition.

### Exemple 3 : Exemple de composition contenant le métronidazole à 1% (m/m).

### a) Lotion à 1 % (m/m):

| Ingrédients | Formule (% m/m) |
|---|---|
| Métronidazole | 1,00 |
| Niacinamide | 2,85 |
| Propylène glycol | 5,00 |
| Macrogol 400 (PEG 400) | 5,00 |
| Alcool benzylique | 1,30 |
| Glycérine | 5,00 |
| Alcool stéarylique | 2,00 |
| Huile minérale | 6,00 |
| Carbomer 981 NF | 0,15 |
| Arlacel 165 FL | 3,00 |
| Sorbate de potassium | 0,20 |
| Cyclométhicone | 4,00 |
| Steareth 21 | 3,00 |
| Hydroxyde de sodium 10% | Qsp pH 5,0 ± 0,5 |
| Eau purifiée | Qsp 100,00 |

La lotion est préparée selon le mode opératoire suivant :
Phase aqueuse :
   1) Dans un bécher, peser l'eau et introduire sous agitation à la défloculeuse ( 300 tr/min) la niacinamide, le Macrogol 400, le propylène glycol et le Métronidazole.
   2) Après totale dissolution du Métronidazole, ajouter la glycérine, le Carbomer 981 et le Steareth 21
   3) Chauffer la phase aqueuse à 70°C
Phase grasse :
   1) Dans un bécher, peser l'alcool stéarylique, l'huile minérale et l'Arlacel 165FL
   2) Chauffer à 70°C et agiter à la défloculeuse ( 300 tr/min)

### Emulsification :

1) Introduire doucement la phase grasse dans la phase aqueuse sous agitation forte (900 tr/min) à l'aide d'un rotor stator en maintenant la température à 70°C
2) Après introduction, laisser sous agitation pendant 10 minutes
3) Laisser refroidir à température ambiante sous agitation plus faible (300 tr/min) et introduire la cyclométhicone, l'alcool benzylique et le sorbate de potassium
4) Ajuster le pH avec une solution d'hydroxyde de sodium à 10% (m/m) si nécessaire
5) Compléter en eau et homogénéiser si évaporation de l'eau lors de l'émulsification

### b) Gel à 1% (m/m) :

| Ingrédients | Formule (% m/m) |
|---|---|
| Métronidazole | 1,00 |
| Niacinamide | 2,85 |
| Propylène glycol | 5,00 |
| Macrogol 400 (PEG 400) | 5,00 |
| Edetate disodique (EDTA) | 0,10 |
| Carbomer 980NF | 0,50 |
| Méthyl parahydroxybenzoate | 0,15 |
| Propyl parahydroxybenzoate | 0,05 |
| Hydroxyde de sodium 10% | Qsp pH 5,0 ± 0,5 |
| Eau purifiée | Qsp 100,00 |

Le gel est préparé selon le mode opératoire suivant :
1) Dans un bécher, peser l'eau et introduire sous agitation à la défloculeuse ( 300 tr/min) la niacinamide, le Macrogol 400, le propylène glycol et le Métronidazole.
2) Après totale dissolution du Métronidazole, ajouter le Carbomer 980NF et l'EDTA, chauffer à 60°C et homogénéiser à la défloculeuse (600 tr/min)
3) Laisser refroidir à température ambiante et ajouter les parabènes, homogénéiser jusqu'à totale dissolution (600 tr/min)
4) Ajuster le pH à l'aide d'une solution d'hydroxyde de sodium à 10% (m/m) si nécessaire
5) Compléter en eau et homogénéiser si évaporation

### c) Crème à 1 % (m/m) :

| Ingrédients | Formule (% m/m) |
|---|---|
| Métronidazole | 1,00 |
| Niacinamide | 2,85 |
| Propylène glycol | 5,00 |
| Macrogol 400 (PEG 400) | 5,00 |
| Alcool benzylique | 2,20 |
| Isopropyl myristate | 2,00 |
| Glycérine | 4,00 |
| Polawax NF | 12,50 |
| Acide lactique 90% | Qsp pH 5,0 ± 0,5 |
| Eau purifiée | Qsp 100,00 |

La crème est préparée selon le mode opératoire suivant :
Phase aqueuse :
   1) Dans un bécher, peser l'eau et introduire sous agitation à la défloculeuse (300 tr/min) la niacinamide, le Macrogol 400, le propylène glycol et le Métronidazole.
   2) Après totale dissolution du Métronidazole, ajouter la glycérine
   3) Chauffer la phase aqueuse à 70°C
Phase grasse :
   1) Dans un bécher, peser l'isopropyl myristate et le Polawax NF
   2) Chauffer à 70°C et agiter à la défloculeuse ( 300 tr/min)
Emulsification :
   1) Introduire doucement la phase grasse dans la phase aqueuse sous agitation forte (900 tr/min) à l'aide d'un rotor stator en maintenant la température à 70°C
   2) Après introduction, laisser sous agitation pendant 10 minutes
   3) Laisser refroidir à température ambiante sous agitation plus faible (300 tr/min) et introduire l'alcool benzylique
   4) Ajuster le pH avec une solution d'acide lactique à 90% si nécessaire
   5) Compléter en eau et homogénéiser si évaporation de l'eau lors de l'émulsification

### d) Spray à 1 % (m/m):

| Ingrédients | Formule (% m/m) |
|---|---|
| Métronidazole | 1,00 |
| Niacinamide | 2,85 |
| Propylène glycol | 5,00 |
| Macrogol 400 (PEG 400) | 5,00 |
| Gomme xanthane | 1,00 |
| Alcool benzylique | 2,00 |
| Ethanol | 30,00 |
| Eau purifiée | Qsp 100,00 |

Le spray est préparé selon le mode opératoire suivant :
1) Dans un bécher, peser l'eau et introduire sous agitation à la défloculeuse ( 300 tr/min) la niacinamide, le Macrogol 400, le propylène glycol et le Métronidazole
2) Après totale dissolution du Métronidazole, ajouter la gomme xanthane, chauffer à 60°C et laisser homogénéiser (600 tr/min)
3) Après totale dissolution de la gomme xanthane, laisser refroidir à température ambiante et ajouter l'éthanol et l'alcool benzylique
4) Homogénéiser jusqu'à l'obtention d'une solution limpide (300 tr/min)

### Exemples 4 : Exemples de compositions contenant le métronidazole à des concentrations supérieures à 1% (m/m):

### a) Gel à 1,5% (m/m):

| Ingrédients | Formule (% m/m) |
|---|---|
| Métronidazole | 1,50 |
| Niacinamide | 5,70 |
| Propylène glycol | 5,00 |
| Macrogol 400 (PEG 400) | 5,00 |
| Edetate disodique (EDTA) | 0,10 |
| Carbomer 940 | 0,60 |
| Méthyl parahydroxybenzoate | 0,15 |
| Propyl parahydroxybenzoate | 0,05 |
| Hydroxyde de sodium 10% | Qsp pH 5,0 ± 0,5 |
| Eau purifiée | Qsp 100,00 |

Le gel à 1,5% (m/m) est préparé selon le même protocole que le gel à 1% (m/m).

### b) Mousse à 1,5% (m/m):

| Ingrédients | Formule (% m/m) |
|---|---|
| Métronidazole | 1,50 |
| Niacinamide | 5,70 |
| Propylène glycol | 5,00 |
| Macrogol 400 (PEG 400) | 5,00 |
| Méthylcellulose | 0,30 |
| Gomme xanthane | 0,30 |
| PEG-40 stéarate | 3,00 |
| Polysorbate 80 | 1,00 |
| Monostéarate de glycéryle | 0,50 |
| Méthylparabène | 0,15 |
| Propylparabène | 0,05 |
| Phénoxyéthanol | 1,00 |
| Huile minérale | 6,00 |
| Acide stéarique | 1,00 |
| Mygliol | 6,00 |
| Eau purifiée | 55,7 |
| Gaz propulseur | Qsp 100,00 |

La mousse est préparée selon le mode opératoire suivant :
Phase aqueuse :
   1) Dans un bécher, peser l'eau et introduire sous agitation à la défloculeuse ( 300 tr/min) la niacinamide, le Macrogol 400, le propylène glycol et le Métronidazole
   2) Après totale dissolution du Métronidazole, chauffer la phase aqueuse à 70°C et ajouter la gomme xanthane et la méthylcellulose, le PEG-40 stéarate, le polysorbate 80 et le monostéarate de glycéryle
   3) En maintenant la température et l'agitation, ajouter le méthylparabène
Phase grasse :
   1) Dans un bécher, peser l'acide stéarique, l'huile minérale
   2) Laisser fondre au bain marie à 70°C et homogénéiser en maintenant l'agitation
Emulsification :
   1) Introduire doucement la phase grasse dans la phase aqueuse sous agitation forte (900 tr/min) à l'aide d'un rotor stator en maintenant la température à 70°C
   2) Laisser refroidir à température ambiante sous agitation plus faible (300 tr/min) jusqu'à une température inférieure à 50°C
   3) Ajouter le phénoxyéthanol et laisser refroidir à température ambiante sous agitation douce
   4) Compléter en eau et homogénéiser si évaporation de l'eau lors de l'émulsification

### Conditionnement :

L'émulsion huile dans eau ainsi obtenue est introduite dans un conditionnement aérosol. Après obturation du récipient, le gaz propulseur est introduit.

## Revendications

1. Procédé de solubilisation du métronidazole, **caractérisé en ce qu'**il comprend l'étape consistant à mélanger en phase aqueuse ledit métronidazole avec de la niacinamide ou ses sels, et au moins deux co-solvants glycoliques, en absence de cyclodextrine.

2. Procédé de solubilisation selon la revendication 1, **caractérisé en ce que** la proportion de métronidazole, en masse par rapport à la masse totale de la solution, est supérieure ou égale à 0.75%.

3. Procédé de solubilisation selon l'une des revendications 1 à 2, **caractérisé en ce que** la proportion de métronidazole, en masse par rapport à la masse totale de la solution, est supérieure ou égale à 1 %.

4. Procédé de solubilisation selon l'une quelconque des revendications 1 à 3, **caractérisé en ce que** le rapport molaire niacinamide / métronidazole est strictement inférieur à 9.

5. Procédé de solubilisation selon l'une quelconque des revendications 1 à 4, **caractérisé en ce que** le rapport molaire niacinamide / métronidazole est inférieur ou égal à 5,5.

6. Procédé de solubilisation selon l'une quelconque des revendications 1 à 5 **caractérisé en ce que** les co-solvants glycoliques sont le propylène glycol et au moins un polyéthylène glycol.

7. Procédé de solubilisation selon l'une quelconque des revendications 1 à 6, **caractérisé en ce que** le polyéthylène glycol est le PEG 400.

8. Procédé de solubilisation selon l'une quelconque des revendications 1 à 7, **caractérisé en ce qu'**on utilise deux co-solvants glycoliques.

9. Procédé de solubilisation selon la revendication 8, **caractérisé en ce que** les cosolvants glycoliques sont présents dans un rapport massique de 1 :1.

10. Procédé de solubilisation selon l'une quelconque des revendications 1 à 9, **caractérisé en ce qu'**il comprend les étapes suivantes :
a) préparation d'une solution de niacinamide dans de l'eau, jusqu'à dissolution totale de la niacinamide,
b) introduction dans la solution obtenue en a) des co-solvants glycoliques,
c) après homogénéisation de la solution obtenue en b), ajout d'une quantité définie de métronidazole,
d) après dissolution totale du métronidazole, filtration de la solution obtenue.

11. Solution aqueuse de métronidazole, **caractérisée en ce qu'**elle est susceptible d'être obtenue selon le procédé décrit dans l'une des revendications 1 à 10.

12. Solution aqueuse comprenant du métronidazole et de la niacinamide susceptible d'être obtenue selon le procédé décrit dans l'une des revendications 1 à 10, **caractérisée en ce qu'**elle comprend au moins deux co-solvants glycoliques.

13. Solution selon la revendication 12, **caractérisée en ce que** le métronidazole est solubilisé et que la solution est exempte de cyclodextrine.

14. Solution selon l'une des revendications 12 ou 13, **caractérisée en ce qu'**elle comprend :
1) du métronidazole en proportion supérieure ou égale à 1%,
2) de la niacinamide en proportion telle que le rapport molaire niacinamide / métronidazole est strictement inférieur à 9,
3) du propylène glycol et un polyéthylène glycol dans un rapport massique de 1 :1.

15. Solution selon l'une des revendications 12 à 14, **caractérisée en ce que** le rapport molaire niacinamide / métronidazole est inférieur ou égal à 5,5.

16. Solution selon l'une des revendications 12 à 15, **caractérisée en ce qu'**elle comprend du propylène glycol et du polyéthylène glycol 400.

17. Composition pharmaceutique, **caractérisée en ce qu'**elle comprend la solution selon l'une quelconque des revendications 11 à 16.

18. Utilisation d'une association de niacinamide et d'au moins deux co-solvants glycoliques pour solubiliser le métronidazole dans une composition comprenant une phase aqueuse substantiellement exempte de cyclodextrine.

19. Utilisation d'une solution selon l'une quelconque des revendications 11 à 16 pour la fabrication d'un médicament destiné au traitement d'une affection dermatologique.

20. Utilisation selon la revendication 19 pour la fabrication d'un médicament destiné au traitement de la rosacée.

21. Utilisation d'une composition selon la revendication 17 pour la fabrication d'un médicament destiné au traitement d'une affection dermatologique.

22. Utilisation selon la revendication 21 pour la fabrication d'un médicament destiné au traitement de la rosacée.

## Claims

1. Process for solubilizing metronidazole, **characterized in that** it comprises the step consisting in mixing, in an aqueous phase, said metronidazole with niacinamide or its salts, and at least two glycolic cosolvents, in the absence of cyclodextrin.

2. Solubilization process according to Claim 1, **characterized in that** the proportion of metronidazole, by mass relative to the total mass of the solution, is greater than or equal to 0.75%.

3. Solubilization process according to either of Claims 1 and 2, **characterized in that** the proportion of metronidazole, by mass relative to the total mass of the solution, is greater than or equal to 1%.

4. Solubilization process according to any one of Claims 1 to 3, **characterized in that** the niacinamide/metronidazole molar ratio is strictly less than 9.

5. Solubilization process according to any one of Claims 1 to 4, **characterized in that** the niacinamide/metronidazole molar ratio is less than or equal to 5.5.

6. Solubilization process according to any one of Claims 1 to 5, **characterized in that** the glycolic cosolvents are propylene glycol and at least one polyethylene glycol.

7. Solubilization process according to any one of Claims 1 to 6, **characterized in that** the polyethylene glycol is PEG 400.

8. Solubilization process according to any one of Claims 1 to 7, **characterized in that** two glycolic cosolvents are used.

9. Solubilization process according to Claim 8, **characterized in that** the glycolic cosolvents are present in a ratio by mass of 1:1.

10. Solubilization process according to any one of Claims 1 to 9, **characterized in that** it comprises the following steps:
a) preparation of a solution of niacinamide in water, until complete dissolution of the niacinamide is obtained,
b) introduction of the glycolic cosolvents into the solution obtained in a),
c) after homogenization of the solution obtained in b), addition of a defined amount of metronidazole,
d) after complete dissolution of the metronidazole, filtration of the solution obtained.

11. Aqueous solution of metronidazole, **characterized in that** it can be obtained according to the process described in one of Claims 1 to 10.

12. Aqueous solution comprising metronidazole and niacinamide, which can be obtained according to the process described in one of Claims 1 to 10, **characterized in that** it comprises at least two glycolic cosolvents.

13. Solution according to Claim 12, **characterized in that** the metronidazole is solubilized and **in that** the solution is free of cyclodextrin.

14. Solution according to either of Claims 12 and 13, **characterized in that** it comprises:
1) metronidazole in a proportion of greater than or equal to 1%,
2) niacinamide in a proportion such that the niacinamide/metronidazole molar ratio is strictly less than 9,
3) propylene glycol and a polyethylene glycol in a ratio by mass of 1:1.

15. Solution according to one of Claims 12 to 14, **characterized in that** the niacinamide/metronidazole molar ratio is less than or equal to 5.5.

16. Solution according to one of Claims 12 to 15, **characterized in that** it comprises propylene glycol and polyethylene glycol 400.

17. Pharmaceutical composition, **characterized in that** it comprises the solution according to any one of Claims 11 to 16.

18. Use of a combination of niacinamide and of at least two glycolic cosolvents for solubilizing metronidazole in a composition comprising an aqueous phase substantially free of cyclodextrin.

19. Use of a solution according to any one of Claims 11 to 16, in the manufacture of a medicament for use in the treatment of a dermatological condition.

20. Use according to Claim 19, in the manufacture of a medicament for use in the treatment of rosacea.

21. Use of a composition according to Claim 17, in the manufacture of a medicament for use in the treatment of a dermatological condition.

22. Use according to Claim 21, in the manufacture of a medicament for use in the treatment of rosacea.

## Patentansprüche

1. Verfahren zur Solubilisierung von Metronidazol, **dadurch gekennzeichnet, dass** es einen Schritt umfasst, der darin besteht, das Metronidazol in wässriger Phase mit Niacinamid oder seinen Salzen und mindestens zwei Glycol-Colösemitteln in Abwesenheit von Cyclodextrin zu vermischen.

2. Verfahren zur Solubilisierung nach Anspruch 1, **dadurch gekennzeichnet, dass** der auf die Masse bezogene Anteil von Metronidazol, bezogen auf die Gesamtmasse der Lösung, größer oder gleich 0,75 % ist.

3. Verfahren zur Solubilisierung nach einem der Ansprüche 1 bis 2, **dadurch gekennzeichnet, dass** der auf die Masse bezogene Anteil von Metronidazol, bezogen auf die Gesamtmasse der Lösung, größer oder gleich 1 % ist.

4. Verfahren zur Solubilisierung nach einem der Ansprüche 1 bis 3, **dadurch gekennzeichnet, dass** das Molverhältnis Niacinamid/ Metronidazol immer kleiner 9 ist.

5. Verfahren zur Solubilisierung nach einem der Ansprüche 1 bis 4, **dadurch gekennzeichnet, dass** das Molverhältnis Niacinamid/ Metronidazol kleiner oder gleich 5,5 ist.

6. Verfahren zur Solubilisierung nach einem der Ansprüche 1 bis 5, **dadurch gekennzeichnet, dass** es sich bei den Glycol-Colösemitteln um Propylenglycol und mindestens ein Polyethylenglycol handelt.

7. Verfahren zur Solubilisierung nach einem der Ansprüche 1 bis 6, **dadurch gekennzeichnet, dass** das Polyethylenglycol das PEG 400 ist.

8. Verfahren zur Solubilisierung nach einem der Ansprüche 1 bis 7, **dadurch gekennzeichnet, dass** zwei Glycol-Colösemittel verwendet werden.

9. Verfahren zur Solubilisierung nach Anspruch 8, **dadurch gekennzeichnet, dass** die Glycol-Colösemittel in einem Masseverhältnis von 1:1 vorliegen.

10. Verfahren zur Solubilisierung nach einem der Ansprüche 1 bis 9, **dadurch gekennzeichnet, dass** es die folgenden Schritte umfasst:
a) Herstellen einer Lösung von Niacinamid in Wasser, bis das Niacinamid vollständig gelöst ist,
b) Einbringen der Glycol-Colösemittel in die in a) gebildete Lösung,
c) Zugeben einer definierten Menge des Metronidazols nach dem Homogenisieren der in b) erhaltenen Lösung,
d) Filtrieren der erhaltenen Lösung nach dem vollständigen Lösen des Metronidazols.

11. Wässrige Lösung von Metronidazol, **dadurch gekennzeichnet, dass** sie nach dem in einem der Ansprüche 1 bis 10 beschriebenen Verfahren erhältlich ist.

12. Wässrige Lösung, die Metronidazol und Niacinamid enthält und nach dem in einem der Ansprüche 1 bis 10 beschriebenen Verfahren erhältlich ist, **dadurch gekennzeichnet, dass** sie mindestens zwei Glycol-Colösemittel enthält.

13. Lösung nach Anspruch 12, **dadurch gekennzeichnet, dass** das Metronidazol gelöst ist und die Lösung kein Cyclodextrin enthält.

14. Lösung nach einem der Ansprüche 12 oder 13, **dadurch gekennzeichnet, dass** sie enthält:
a) Metronidazol in einer Menge von 1 % oder darüber,
b) Niacinamid in einem solchen Mengenanteil, dass das Molverhältnis Niacinamid/ Metronidazol immer kleiner 9 ist;
c) Propylenglycol und ein Polyethylenglycol in einem Masseverhältnis von 1:1.

15. Lösung nach einem der Ansprüche 12 bis 14, **dadurch gekennzeichnet, dass** das Molverhältnis Niacinamid/Metronidazol kleiner oder gleich 5,5 ist.

16. Lösung nach einem der Ansprüche 12 bis 15, **dadurch gekennzeichnet, dass** sie Propylenglycol und Polyethylenglycol 400 enthält.

17. Pharmazeutische Zusammensetzung, **dadurch gekennzeichnet, dass** sie die Lösung nach einem der Ansprüche 11 bis 16 enthält.

18. Verwendung einer Kombination von Niacinamid und mindestens zwei Glycol-Colösemittel zur Solubilisierung von Metronidazol in einer Zusammensetzung, die eine wässrige Phase enthält, die im Wesentlichen frei von Cyclodextrin ist.

19. Verwendung einer Lösung nach einem der Ansprüche 11 bis 16 für die Herstellung eines Arzneimittels, das für die Behandlung einer dermatologischen Erkrankung vorgesehen ist.

20. Verwendung nach Anspruch 19 für die Herstellung eines Arzneimittels, das für die Behandlung von Rosacea vorgesehen ist.

21. Verwendung einer Zusammensetzung nach Anspruch 17 für die Herstellung eines Arzneimittels, das für die Behandlung einer dermatologischen Erkrankung vorgesehen ist.

22. Verwendung nach Anspruch 21 für die Herstellung eines Arzneimittels, das für die Behandlung von Rosacea vorgesehen ist.
